Europäisches Patentamt

European Patent Office

Office européen des brevets

(11) Veröffentlichungsnummer: **0 324 811 B1**

# (12) EUROPÄISCHE PATENTSCHRIFT

(45) Veröffentlichungstag der Patentschrift :
**01.04.92 Patentblatt 92/14**

(21) Anmeldenummer : **88905673.5**

(22) Anmeldetag : **30.06.88**

(86) Internationale Anmeldenummer :
**PCT/DE88/00399**

(87) Internationale Veröffentlichungsnummer :
**WO 89/00080 12.01.89 Gazette 89/02**

(51) Int. Cl.⁵ : **B01J 4/00, B01F 3/02,**
**// C23C16/44**

(54) **GASEINLASS FÜR EINE MEHRZAHL VERSCHIEDENER REAKTIONSGASE IN REAKTIONSGEFÄSSE.**

(30) Priorität : **30.06.87 DE 3721637**

(43) Veröffentlichungstag der Anmeldung :
**26.07.89 Patentblatt 89/30**

(45) Bekanntmachung des Hinweises auf die
Patenterteilung :
**01.04.92 Patentblatt 92/14**

(84) Benannte Vertragsstaaten :
**AT BE CH DE FR GB IT LI LU NL SE**

(56) Entgegenhaltungen :
**DE-A- 1 557 203**
**FR-A- 648 275**

(56) Entgegenhaltungen :
**Extended Abstracts, Band 84 Nr. 2, October**
**1984,(New Orleans, Louisiana, US) B.A. Ca-**
**pron et al.: "Silicon nitride deposition in a**
**vertical flow reactor", Seite 738, Zusammen-**
**fassung Nr. 505**
**IBM Technical Disclosure Bulletin, Band 26,**
**Nr.7B, Dezember 1983, IBM Corp. (New York,**
**US), J.P. Gallagher et al.: "Gas injector for**
**nitride deposition using 2% silane gas2,Seiten**
**3577-3578**

(73) Patentinhaber : **AIXTRON GMBH**
**Jülicher Strasse 336**
**W-5100 Aachen (DE)**

(72) Erfinder : **JÜRGENSEN, Holger**
**Melatenerstr. 56**
**W-5100 Aachen (DE)**

(74) Vertreter : **Münich, Wilhelm, Dr. et al**
**Kanzlei Münich, Steinmann, Schiller**
**Willibaldstrasse 36/38**
**W-8000 München 21 (DE)**

EP 0 324 811 B1

## Beschreibung

### Technisches Gebiet

Die Erfindung bezieht sich auf einen Gaseinlaß für eine Mehrzahl verschiedener Reaktionsgase in Reaktionsgefäße, in denen die Gase mit hoher Strömungsgeschwindigkeit strömen, mit Zuführleitungen, deren Querschnitt wesentlich kleiner als der des Reaktionsgefäßes ist.

### Stand der Technik

Derartige Gaseinlässe werden beispielsweise bei MOCVD-Reaktoren, aber auch bei anderen Reaktoren zur Halbleiter-Herstellung benötigt. Dabei stellt sich allgemein das Problem, daß ein oder mehrere Gasströme, die durch eine Versorgungsleitung mit relativ geringem Querschnitt zugeführt werden, möglichst homogen und ohne Totvolumina auf einen großen Querschnitt aufgeweitet werden soll. Dies ist insbesondere dann von Bedeutung, wenn durch "Umschalten" der Gasströme möglichst "scharfe" Schichtbegrenzungen erzeugt werden sollen.

Ein Gaseinlaß für ein Reaktionsgas in ein Reaktionsgefäß ist aus IBM, Technical Disclosure Bulletin, Bd. 26, Nr. 7b, S. 3577-3578 (1983) bekannt, bei dem ein Gas mit hoher Strömungsgeschwindigkeit aus einer Zuführleitung, deren Querschnitt wesentlich kleiner als der des Reaktionsgefäßes ist, dadurch in das Reaktionsgefäß homogen eingeleitet wird, daß die Zuführleitung auf den Querschnitt des Reaktionsgefäßes aufgeweitet wird. Diese Lösung ist jedoch dann nicht anwendbar, wenn eine Mehrzahl verschiedener Reaktionsgase in Reaktionsgefäße eingeleitet werden soll.

### Darstellung der Erfindung

Der Erfindung liegt die Aufgabe zugrunde, einen Gaseinlaß für eine Mehrzahl von Reaktionsgasen in Reaktionsgefäße gemäß dem Oberebgriff des Anspruchs 1 derart weiterzubilden, daß ein oder mehrere Gasströme möglichst homogen und ohne Totvolumina auf einen vergleichsweise großen Querschnitt aufgeweitet werden können.

Eine erfindungsgemäße Lösung dieser Aufgabe ist mit ihren Weiterbildungen in den Patentansprüchen gekennzeichnet.

Erfindungsgemäß weist der Gaseinlaß einen Teil mit conicoider Innenkontur auf, d.h. mit einer Innenkontur, deren Schnittlinien mit einer Ebene Kegelschnitte sind. Der Querschnitt dieses Teils ist dem Querschnitt des Reaktionsgefäßes angepaßt. Ferner ist das Teil an einem Ende des durchströmten Reaktionsgefäßes angeordnet. Die einzelnen Zuführleitungen münden in etwa im Brennpunkt des Teils mit conicoider Innenkontur, wobei die Gasaustrittsöffnungen der Zuführleitungen auf den Scheitelpunkt des conicoiden Teils gerichtet sind.

Hierdurch wird der "dünne" Gasstrahl, der mit hoher Geschwindigkeit aus den Gasaustrittsöffnungen der Zuführleitungen austritt, von der conicoiden Innenkontur "praktisch nach den Gesetzen der geometrischen Optik" homogen und ohne Totvolumina aufgeweitet. Weiterbildungen der Erfindung sind in den Unteransprüchen gekennzeichnet:

Durch die im Anspruch 2 gekennzeichnete sternförmige Anordnung der Zuführleitungen ist das (gleichzeitige und/ oder "serielle") Einleiten mehrerer Gassorten möglich, ohne daß die Homogenität des aufgeweiteten Gasstroms gestört würde.

Für praktische Zwecke wird eine mehr als ausreichende Homogenität des aufgeweiteten Gasstrahls erhalten, wenn die Innenkontur des conicoiden Teils, d.h. die conicoide Form durch eine Halbkugel angenähert ist.

Durch die im Anspruch 4 gekennzeichnete Maßnahme wird eine Zerstäubung und damit eine weitere Homogenisierung des Gasstroms über den Querschnitt des Reaktors erreicht.

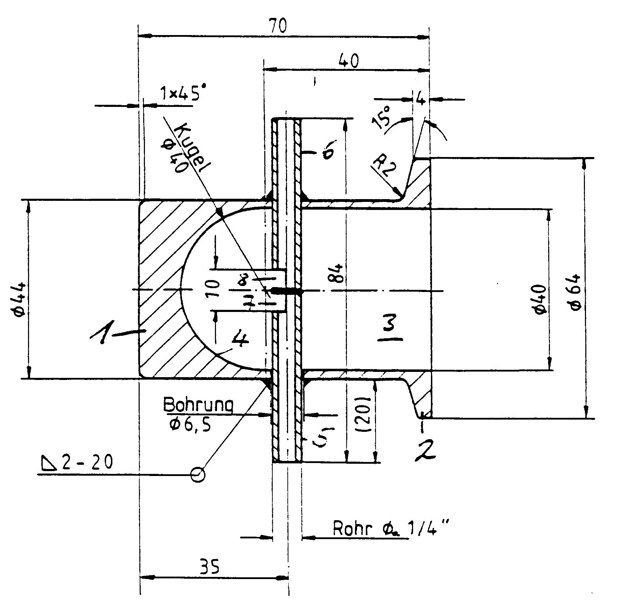

### Kurze Beschreibung der Zeichnung

Die Erfindung wird nachstehend anhand eines Ausführungsbeispiels unter Bezugnahme auf die Zeichnung näher beschrieben, deren einzige Figur einen Querschnitt durch einen erfindungsgemäßen Gaseinlaß zeigt.

Ausdrücklich soll darauf hingewiesen werden, daß die in der Zeichnung angegebenen Maße in mm exemplarisch zu verstehen sind, und die Erfindung nicht einschränken.

### Beschreibung eines Ausführungsbeispiels

Der erfindungsgemäße Gaseinlaß weist einen Grundkörper 1 auf, an dem sich ein Flansch 2 befindet. Der Flansch 2 dient zur Verbindung des Grundkörpers 1 mit einem nicht dargestellten Reaktor, beispielsweise einem Reaktor, wie er in der am gleichen Tage eingereichten Patentanmeldung "Quarzglasreaktor für MOCVD-Anlagen" beschrieben ist.

Die in dem Grundkörper 1 vorgesehene Ausnehmung 3 hat eine "hintere" Abschlußfläche 4, deren Form bei dem gezeigten Ausführungsbeispiel halbkugelförmig ist.

Ferner sind Gaszuführleitungen vorgesehen, die sternförmig angeordnet sind, und von denen in der Zeichnung lediglich zwei Leitungen 5 und 6 dargestellt sind. Die Zuführleitungen 5 und 6 weisen Austrittsöffnungen 7 und 8 auf, die sich in etwa im Mittelpunkt der Halb-Kugelfläche 4 befinden. Die Kanten der Austrittsöffnungen sind "gebrochen", so daß der austretende Gasstrom teilweise "zerstäubt" wird.

Der erfindungsgemäße Gaseinlaß arbeitet wie folgt:

Die aus den Austrittsöffnungen 7 bzw. 8 mit hoher Geschwindigkeit austretenden Gasströme werden durch die Kantenstruktur der Öffnungen zum Teil zerstäubt und treffen auf einen Bereich der Fläche 4 auf. An dieser Fläche 4 wird der mit hoher Geschwindigkeit austretende Gasstrom "praktisch nach den Gesetzen der geometrischen Optik" reflektiert, so daß er homogen aufgeweitet und durch den "im Gegenstrom" aus den Öffnungen 7 und 8 austretenden , Gasstrom weiter zerstäubt durch die Öffnung des Flansches 2 in einen nicht dargestellten Reaktor austritt.

Vorstehend ist die Erfindung anhand eines Ausführungsbeispiels ohne Beschränkung des allgemeinen Erfindungsgedankens beschrieben worden, innerhalb dessen selbstverständlich die verschiedensten Modifikationen möglich sind:

So kann beispielsweise anstelle einer Kugelfläche auch eine Kegelschnittfläche (conicoide Innenkontur), beispielsweise ein Paraboloid verwendet werden. Diese aus der geometrischen Optik bekannte vorteilhafte Weiterentwicklung sphärischer Flächen bringt jedoch im vorliegenden Falle nur geringe Vorteile.

Die Zahl der Gaseinlaß-Leitungen kann beliebig erhöht werden. Durch die sternförmige Anordnung ergeben sich für alle Gasströme die selben Verhältnisse und insbesondere eine homogene Gasverteilung über den Querschnitt.

Der erfindungsgemäße Gaseinlaß ist damit nicht nur zum aufeinanderfolgendem Einlassen unterschiedlicher Gasströme, die schnell "umgeschaltet" werden sollen, sondern auch zum gleichzeitigen Einlassen mehrerer Gasströme, die homogen aufgeweitet und gut durchmischt werden sollen, geeignet.

## Patentansprüche

1. Gaseinlaß für eine Mehrzahl verschiedener Reaktionsgase in Reaktionsgefäße, in denen die Gase mit hoher Strömungsgeschwindigkeit strömen, mit Zuführleitungen, deren Querschnitt wesentlich kleiner als der des Reaktionsgefäßes ist, **gekennzeichnet** durch die Kombination folgender Merkmale:
– der Gaseinlaß weist einen Teil mit conicoider Innenkontur, d.h. einer Innenkontur, deren Schnitte mit einer Ebene Kegelschnitte sind, auf, dessen Querschnitt dem Querschnitt des Reaktionsgefäßes angepaßt ist und der an einem Ende des durchströmten Reaktionsgefäßes angeordnet ist
– die einzelnen Zuführleitungen münden in etwa im Brennpunkt der conicoiden Innenkonfur,
– die Gasaustrittsöffnungen der Zuführleitungen sind auf den Scheitelpunkt dieses Teils gerichtet.

2. Gaseinlaß nach Anspruch 1, dadurch **gekennzeichnet**, daß die Zuführleitungen "sternförmig" angeordnet sind.

3. Gaseinlaß nach Anspruch 1 oder 2, dadurch **gekennzeichnet**, daß die Innenkontur des Teils halbkugel-förmig ist.

4. Gaseinlaß nach einem der Ansprüche 1 bis 3, dadurch **gekennzeichnet**, daß die Gasaustrittsöffnungen der Zuführleitungen den Gasstrom zerstäuben.

## Claims

1. Gas inlet for a plurality of various reaction gases in reaction vessels in which the gases flow at a high velocity of flow, which comprises feed lines having a cross-sectional area substantially smaller than the cross-sectional area of the reaction vessel, **characterized** by the combination of the following features:
– the gas inlet comprises one section having a conicoidal inner contour, i.e. an inner contour whose lines of intersection with a plane are ellipses, parabolas or trajectories,
– the individual feed lines open out approximately in the focal point of the conicoidal inner contour,

– the gas discharge openings of the feed lines are directed towards the apex of that section.

2. Gas inlet according to Claim 1, **characterized** in that said feed lines are disposed in a "star-shaped" configuration.

3. Gas inlet according to Claim 1 or 2, **characterized** in that the inner contour of said section is hemispherical.

4. Gas inlet according to any of Claims 1 to 3, **characterized** in that said gas discharge openings of the feed lines vaporize or spray said gas flow.


## Revendications

1. Admission d'une pluralité de gaz de réaction dans des réacteurs dans lesquels les gaz passent à une grande vitesse d'écoulement, qui comprend des conduites d'amenée à une section transversale essentiellement plus petite que la section transversale du réacteur, **caractérisé** par la combinaison des caractéristiques suivantes:

– l'admission de gaz comprend une partie à un contour intérieur conicoïde, c.a.d. un contour intérieur dont les intersections avec un plan constituent des sections coniques, la section transversale de ladite admission étant adaptée à la section transversale du réacteur et l'admission étant disposée à une extrémité du réacteur traversé;

– les conduites d'amenée individuelles débouchent environ au foyer du contour intérieur conicoïdal,

– les orifices de sortie de gaz des conduites d'amenée sont dirigés vers l'apex de cette partie.

2. Admission de gaz selon la revendication 1, **caractérisé** en ce que les conduites d'amenée sont disposées "en forme d'étoile".

3. Admission de gaz selon la revendication 1 ou 2, **caractérisé** en ce que le contour intérieur de la partie est hémisphérique.

4. Admission de gaz selon une quelconque des revendications 1 à 3 , **caractérisé** en ce que les orifices de sortie de gaz desdites conduites d'amenée atomisent le courant de gaz.